Europäisches Patentamt

(19) European Patent Office

Office européen des brevets

(11) Publication number: **0 116 635**
**B1**

(12) **EUROPEAN PATENT SPECIFICATION**

(45) Date of publication of patent specification: **20.08.86**

(51) Int. Cl.⁴: **G 01 N 33/72**

(21) Application number: **83902937.8**

(22) Date of filing: **25.08.83**

(86) International application number:
**PCT/US83/01300**

(87) International publication number:
**WO 84/01032 15.03.84 Gazette 84/07**

(54) **A DIRECT BILIRUBIN ASSAY METHOD.**

(30) Priority: **27.08.82 US 412055**

(43) Date of publication of application:
**29.08.84 Bulletin 84/35**

(45) Publication of the grant of the patent:
**20.08.86 Bulletin 86/34**

(84) Designated Contracting States:
**CH DE FR GB LI NL SE**

(56) References cited:
**EP-A-0 021 598**
**US-A-4 246 133**

**Clinical Chemistry, vol. 25, no. 11, publ.
November 1979, (Easton, Pennsylvania, US),
J.S. NOVROS et al.: "Improved method for
accurate quantitation of total and conjugated
bilirubin in serum", pages 1891-1899**

**R.J.Henry et al.: "Clinical Chemistry, Principles
& Technics", 1974, 2nd edition, Harper & Row
(New York, US), pages 1042-1061**

(73) Proprietor: **BECKMAN INSTRUMENTS, INC.
Executive Office 2500 Harbor Boulevard Box
3100
Fullerton California 92634 (US)**

(72) Inventor: **SHU, Frank, R.
401 South Black Oak Road
Anaheim, CA 92807 (US)**
Inventor: **OLANDER, Glenn, J.
465 Fair Drive, Nr. 203
Costa Mesa, CA 92626 (US)**

(74) Representative: **Wotherspoon, Graham et al
FITZPATRICKS 4 West Regent Street
Glasgow G2 1RS Scotland (GB)**

(56) References cited:
**Chemical Abstracts, vol. 90, no.5, 29 January
1979, (Columbus, Ohio, US), J.B. Landis et al.:
"Kinetics of the reactions of unconjugated and
conjugated bilirubins with p-
diazobenzenesulfonic acid", page 170, abstract
no. 35241q**

Courier Press, Leamington Spa, England.

**Description**

Background of the invention
1. Field of the invention
   This invention relates to a method for assaying direct bilirubin.

2. Description of the prior art
   Direct bilirubin, also known as conjugated bilirubin, is usually assayed by coupling it with a diazo dye in an acidic medium. In a typical Jendrassik-Grof procedure, the dye is prepared by diazotizing sulfanilic acid with sodium nitrite. The azobilirubin formation reaction is carried out in a dilute hydrochloric acid solution. The optical density (O.D.) change is then read at the end of a fixed reaction time, usually between 1 to 5 minutes, depending upon the specific procedure employed, and compared with a bilirubin calibration curve for results. The testing procedure is time consuming and susceptible to error due to the degradation of the unstable diazo reagent.
   Recently, Rizi et al. (1) have proposed a kinetic method for the determination of direct bilirubin. By design, their method is a modification of a one minute end-point procedure. The method of Rizi et al. eliminates the need of sample blank but does not improve the selectivity of the test.
   In an ingenious effort, Landis et al. (2) have developed a rate method which can determine both total and direct bilirubin simultaneously in a single measurement. The drawback to Landis et al.'s method is that it does not allow direct bilirubin to be determined independently. As a result any error in determination of unconjugated bilirubin becomes an error in the measurement of conjugated bilirubin.
   Accordingly, it would be desirable to have a method capable of independently assaying direct bilirubin which also eliminates the reagent degradation problems and the time consuming aspects of the prior art procedure.

Summary of the invention
   In accordance with the present invention, there is provided an independent method for measuring direct bilirubin which eliminates the problems associated with the degradation of the diazo reagent, shortens the time required per test and eliminates the need for a sample blank. More particularly, the method of the instant invention for independently assaying the direct bilirubin content of a sample comprises:
   (a) mixing a sulfanilic acid solution comprising from 0.02 to 0.05 M sulfanilic acid and from 0.02 to 0.15 M hydrochloric acid with a sodium nitrite solution comprising from 0.001 to 0.015 M sodium nitrite to thereby commence a diazotization process, the ratio of the sulfanilic acid solution to the sodium nitrite solution being preselected so as to yield a diazo solution comprising from 0.0001 to 0.001 M diazo dye;
   (b) allowing the diazotization process to proceed to form the diazo solution for at least 15 seconds;
   (c) introducing an aliquot of the sample to be assayed into the dye solution at a volume ratio of from 1:100 to 1:25 to thereby commence an azobilirubin process; and
   (d) measuring the instantaneous rate of azobilirubin formation at 545 nm in a time range of from 12 to 25 seconds after introduction of the aliquot of the sample into the diazo solution.

   Still other features and attendant advantages of the present invention will become apparent to those skilled in the art from a reading of the following detailed description of the preferred embodiment.

Description of the preferred embodiment
   The method of the instant invention for assaying the direct bilirubin content of a sample comprises:
   (a) mixing a sulfanilic acid solution comprising from 0.02 to 0.05 M sulfanilic acid and from 0.03 to 0.15 M hydrochloric acid with a sodium nitrite solution comprising from 0.001 to 0.015 M sodium nitrite to thereby commence a diazotization process, the ratio of the sulfanilic acid solution to the sodium nitrite solution being preselected so as to yield a diazo solution comprising from 0.0001 to 0.001 M diazo dye;
   (b) allowing the diazotization process to proceed to form the diazo solution for at least 15 seconds;
   (c) introducing an aliquot of sample to be assayed into the diazo solution at a volume ratio of from 1:100 to about 1:25 to thereby commence an azobilirubin process; and
   (d) measuring the instantaneous rate of the azobilirubin formation at 545 nm in a time range of from 12 to 25 seconds after introduction of the aliquot of the sample into the diazo solution.

   The ratio of the sulfanilic acid solution to the sodium nitrite solution is preferably preselected so as to yield a diazo solution comprising about 0.0004 M diazo dye.
   The sulfanilic acid solution preferably comprises 0.03 M sulfanilic acid and 0.05 M hydrochloric acid.
   The sodium nitrite solution preferably comprises 0.004 M sodium nitrite.
   The diazotization process is preferably allowed to proceed for 30 seconds, and more preferably, is allowed to proceed for 30 seconds at 37°C.
   In addition, it is preferred that the aliquot of sample be introduced into the diazo solution at a volume ratio of 1:55.
   Furthermore, it is preferred that the instantaneous rate measurement be made 18 seconds after introduction of the aliquot of the sample into the diazo solution.
   A preservative may optionally be incorporated into the sodium nitrite solution. Typical preservatives include, but are not limited to, (ethylenedinitrilo)tetraacetic · acid, tetrasodium salt.
   The sulfanilic acid solution and sodium nitrite solution employed in the method involved in the instant invention can be prepared by any method well known to those skilled in the art.

The following examples are provided for purposes of further illustration only and are not intended to be limitations on the disclosed invention.

Example 1

Solutions of sulfanilic acid (0.03 M sulfanic acid and 0.05 M hydrochloric acid) and sodium nitrite (0.004 M sodium nitrite) were mixed in a 10:1 ratio to yield a diazo solution of 1.1 ml comprising about 0.004 M diazo dye. After 30 seconds of diazotization, a 20 µl aliquot of a serum sample was added to the diazo solution. The instantaneous rate of azobilirubin formation was read 18 seconds after sample addition. This procedure was repeated for a total of 20 serum samples and the data obtained therefrom is set forth in Table I.

Example 2

The same 20 serum samples in Example 1 were also assayed via American Monitor Corporation's modified Jendrassik and Grof procedure. The data obtained therefrom is also set forth in Table I.

TABLE I

Direct bilirubin serum correlation, mg/dl

| Serum sample | Example 1 (instant invention) | Example 2 (prior art) |
|---|---|---|
| 1 | 0.11 | 0.03 |
| 2 | 1.16 | 1.62 |
| 3 | 0.42 | 0.44 |
| 4 | 2.00 | 1.67 |
| 5 | 4.40 | 4.45 |
| 6 | 0.14 | 0.24 |
| 7 | 0.32 | 0.33 |
| 8 | 0.07 | 0.14 |
| 9 | 0.11 | 0.11 |
| 10 | 0.95 | 0.90 |
| 11 | 0.60 | 0.68 |
| 12 | 6.93 | 6.78 |
| 13 | 0.11 | 0.06 |
| 14 | 0.32 | 0.34 |
| 15 | 0.11 | 0.13 |
| 16 | 0.11 | 0.02 |
| 17 | 2.11 | 2.38 |
| 18 | 5.90 | 6.14 |
| 19 | 2.95 | 2.63 |
| 20 | 0.32 | 0.53 |

$$Y_{Ex1}=0.9983\ X_{Ex2}-0.215$$
$$r=0.9959$$

The data set forth in Table I show that the method of the instant invention correlates well (r=0.9959) with a commercially available modified Jendrassik and Grof procedure.

However, since the procedure of the instant invention forms the diazo reagent in situ and because the sulfanilic acid and sodium nitrite solutions are much more stable in comparison to the diazo reagent, the method of this invention enables one to employ solutions which have a shelf-like considerably longer than that of the reagents employed in the prior art (i.e., the solutions employed in this invention have a shelf-life of at least 30 days at room temperature vs. the 4 hour room temperature stability of the diazo reagent employed in the prior art). Accordingly, in practicing the method of the instant invention one gains a substantial amount of time by not having to frequently prepare the reagent employed therein. In addition, the increased stability of the solutions employed in this invention reduces the frequency at which one must calibrate the instrument and thereby improves the accuracy of the results obtained via the assays performed.

Bibliography
1. Rizi et al., *Clin. Chem. 23:*1128 (1977).
2. Landis et al., *Clin. Chem. 24:*1700 (1978).

**Claims**

1. A method for assaying the direct bilirubin content of a sample comprising:

(a) mixing a sulfanilic acid solution comprising from 0.02 to 0.05 M sulfanilic acid and from 0.02 to 0.15 M hydrochloric acid with a sodium nitrite solution comprising from 0.001 to 0.015 M sodium nitrite to thereby commence a diazotization process, the ratio of said sulfanilic acid solution to said sodium nitrite solution being preselected so as to yield a diazo solution comprising from 0.0001 to 0.001 M diazo dye;

(b) allowing said diazotization process to proceed to form said diazo solution for at least 15 seconds;

(c) introducing an aliquot of said sample to be assayed into said diazo solution at a volume ratio of from 1:100 to 1:25 to thereby commence an azobilirubin process; and

(d) measuring the instantaneous rate of azobilirubin formation at 545 nm in a time range of from 12 to 25 seconds after introduction of said aliquot of said sample into said diazo solution.

2. The method of claim 1 wherein the ratio of said sulfanilic acid solution to said sodium nitrite solution is preselected so as to yield said diazo solution comprising 0.004 M diazo dye.

3. The method of claim 1 wherein said sulfanilic acid solution comprises 0.03 M sulfanilic acid and 0.05 M hydrochloric acid.

4. The method of claim 1 wherein said sodium nitrite solution comprises 0.004 M sodium nitrite.

5. The method of claim 1 wherein said diazotization process is allowed to proceed for 30 seconds.

6. The method of claim 1 wherein said diazotization process is allowed to proceed for 30 seconds at 37°C.

7. The method of claim 1 wherein said aliquot of said sample is introduced into said diazo solution at a volume ratio of 1:55.

8. The method of claim 1 wherein said instantaneous rate measurement is made at 18 seconds after introduction of said aliquot of said sample into said diazo solution.

9. The method of claim 1, wherein:

(a) said sulfanilic acid solution comprises 0.03 M sulfanilic acid and 0.05 M hydrochloric acid;

(b) said sodium nitrite solution comprises 0.004 M sodium nitrite;

(c) the ratio of said sulfanilic acid solution to said sodium nitrite solution is preselected so as to yield said diazo solution comprising 0.004 M diazo dye;

(d) said diazotization process is allowed to proceed for 30 seconds;

(e) said aliquot of said sample is introduced into said diazo solution at a volume rate of 1:55; and

(f) said instantaneous rate measurement is made at 18 seconds after introduction of said aliquot of said sample into said diazo solution.

10. The method of claim 9 wherein said diazotization process is allowed to proceed at 37°C.

11. The method of claim 9 wherein said sodium nitrite solution further comprises a preservative.

12. The method of claim 1 wherein:

(a) said sulfanilic acid solution comprises 0.03 M sulfanilic acid and 0.05 M hydrochloric acid;

(b) said sodium nitrite solution comprises 0.004 M sodium nitrite and further comprises a preservative;

(c) the ratio of said sulfanilic acid solution to said sodium nitrite solution is preselected so as to yield said diazo solution comprising 0.004 M diazo dye;

(d) said diazotization process is allowed to proceed for 30 seconds at 37°C;

(e) said aliquot of said sample is introduced into said diazo solution at a volume ratio of 1:55; and

(f) said instantaneous rate measurement is made at 18 seconds after introduction of said aliquot of said sample into said diazo solution.

**Patentansprüche**

1. Verfahren zur analytischen Bestimmung des Direkt-Bilirubin-Gehalts einer Probe, umfassend:

(a) Mischen einer von 0,02 bis 0,05 M Sulfanilsäure und von 0,02 bis 0,15 M Chlorwasserstoffsäure enthaltenden Sulfanilsäurelösung mit einer von 0,001 bis 0,015 M Natriumnitrit enthaltenden Natriumnitritlösung, zur Einleitung eines Diazotierungsprozesses, wobei das Verhältnis der genannten Sulfanilsäurelösung zu der genannten Natriumnitritlösung so vorgewählt ist, daß eine von 0,0001 bis 0,001 M Diazo-Farbstoff enthaltende Diazolösung erhalten wird;

(b) der genannte Diazotierungsprozeß wird wenigstens 15 Sekunden lang zur Bildung der genannten Diazolösung ablaufen gelassen;

(c) Einbringen einer Teilmenge der genannten zu analysierenden Probe in die genannte Diazolösung in einem Volumenverhältnis von 1:100 bis 1:25, zur Auslösung eines Azobilirubin-Prozesses; und

(d) Messen der Momentangeschwindigkeit der Azobilirubinbildung bei 545 nm in einem Zeitbereich von 12 bis 25 Sekunden nach der Einbringung der genannten Teilmenge der Probe in die genannte Diazolösung.

2. Verfahren nach Anspruch 1, dadurch gekennzeichnet, daß das Verhältnis der genannten Sulfanilsäurelösung zu der genannten Natriumnitritlösung so vorgewählt wird, daß die genannte Diazolösung mit einem Diazofarbstoffgehalt von 0,004 M erhalten wird.

3. Verfahren nach Anspruch 1, wobei die Sulfanilsäurelösung 0,03 M Sulfanilsäure und 0,05 M Chlorwasserstoffsäure enthält.

4. Verfahren nach Anspruch 1, wobei die genannte Natriumnitrilösung 0,004 M Natriumnitrit enthält.

5. Verfahren nach Anspruch 1, wobei der genannte Diazotierungsprozeß 30 Sekunden lang ablaufen gelassen wird.

6. Verfahren nach Anspruch 1, bei welchem der genannte Diazotierungsprozeß 30 Sekunden lang bei 37°C ablaufen gelassen wird.

7. Verfahren nach Anspruch 1, bei welchem die genannte Teilmenge der genannten Probe in die genannte Diazolösung in einem Volumenverhältnis von 1:55 eingebracht wird.

8. Verfahren nach Anspruch 1, bei welchem die genannte Messung der Momentan-Geschwindigkeit der Azobilirubinbildung 18 Sekunden nach dem Einbringen der genannten Teilmenge der Probe in die genannte Diazolösung durchgeführt wird.

9. Verfahren nach Anspruch 1, bei welchem

(a) die genannte Sulfanilsäurelösung 0,03 M Sulfanilsäure und 0,05 M Chlor-wasserstoffsäure enthält;

(b) die genannte Natriumnitritlösung 0,004 M Natriumnitrit enthält;

(c) das Verhältnis der genannten Sulfanilsäurelösung zu der genannten Natriumnitritlösung so vorgewählt wird, daß die genannte Diazolösung mit einem Diazofarbstoffgehalt von 0,004 M erhalten wird;

(d) der genannte Diazotierungsprozeß 30 Sekunden lang ablaufen gelassen wird;

(e) die genannte Teilmenge der Probe in die Diazolösung in einem Volumenverhältnis von 1:55 eingebracht wird; und

(f) die Messung der Momentan-Bildungsgeschwindigkeit 18 Sekunden nach der Einbringung der Probenteilmenge in die Diazolösung vorgenommen wird.

10. Verfahren nach Anspruch 9, bei welchem der Diazotierungsprozeß bei 37°C ablaufen gelassen wird.

11. Verfahren nach Anspruch 9, bei welchem die Natriumnitritlösung des weiteren ein Konservierungsmittel enthält.

12. Verfahren nach Anspruch 1, bei welchem

(a) die genannte Sulfanilsäurelösung 0,03 M Sulfanilsäure und 0,05 M Chlorwasserstoffsäure enthält;

(b) die genannte Natriumnitritlösung 0,004 M Natriumnitrit und des weiteren ein Konservierungsmittel enthält;

(c) des Verhältnis der Sulfanilsäurelösung zu der Natriumnitritlösung so vergewählt wird, daß die genannte Diazolösung mit einem Gehalt von 0,004 M Diazofarbstoff erhalten wird;

(d) der genannte Diazotierungsprozeß 30 Sekunden lang bei 37°C ablaufen gelassen wird;

(e) die Probenteilmenge in die Diazolösung mit einem Volumenverhältnis von 1:55 eingebracht wird; und

(f) die Messung der momentanen Bildungsgeschwindigkeit 18 Sekunden lang nach der Einbringung der Probenteilmenge in die Diazolösung vorgenommen wird.

**Revendications**

1. Procédé de titrage de la teneur en bilirubine directe d'un échantillon comprenant:

(a) le mélange d'une solution d'acide sulfanilique comprenant de 0,02 à 0,05 M d'acide sulfanilique et de 0,02 à 0,15 M d'acide chlorhydrique avec une solution de nitrite de sodium comprenant de 0,001 à 0,015 M de nitrite de sodium pour déclencher ainsi un processus de diazotation, le rapport de ladite solution d'acide sulfanilique à ladite solution de nitrite de sodium étant préalablement choisi de manière à obtenir une solution diazoïque comprenant de 0,0001 à 0,001 M de colorant diazoïque;

(b) la poursuite dudit processus de diazotation pour former ladite solution diazoïque pendant au moins 15 secondes;

(c) l'introduction d'une partie aliquote dudit échantillon à titrer dans ladite solution diazoïque dans un rapport en volume de 1:100 à 1:25 pour déclencher ainsi un processus de formation d'azobilirubine; et

(d) la mesure de la vitesse instantanée de formation d'azobilirubine à 545 nm pendant un temps de 12 à 25 secondes après l'introduction de ladite partie aliquote dudit échantillon dans ladite solution diazoïque.

2. Procédé suivant la revendication 1, dans lequel le rapport de ladite solution d'acide sulfanilique à ladite solution de nitrite de sodium est préalablement choisi de manière à obtenir ladite solution diazoïque comprenant 0,004 M de colorant diazoïque.

3. Procédé suivant la revendication 1, dans lequel ladite solution d'acide sulfanilique comprend 0,03 M d'acide sulfanilique et 0,05 M d'acide chlorhydrique.

4. Procédé suivant la revendication 1, dans lequel ladite solution de nitrite de sodium comprend 0,004 M de nitrite de sodium.

5. Procédé suivant la revendication 1, dans lequel ledit processus de diazotation est poursuivi pendant 30 secondes.

6. Procédé suivant la revendication 1, dans lequel ledit processus de diazotation est poursuivi pendant 30 secondes à 37°C.

7. Procédé suivant la revendication 1, dans lequel ladite partie aliquote dudit échantillon est introduite dans ladite solution diazoïque dans un rapport en volume de 1:55.

8. Procédé suivant la revendication 1, dans lequel ladite mesure de la vitesse instantanée est effectuée 18 secondes après l'introduction de ladite partie aliquote dudit échantillon dans ladite solution diazoïque.

9. Procédé suivant la revendication 1, dans lequel:

(a) ladite solution d'acide sulfanilique comprend 0,03 M d'acide sulfanilique et 0,05 M d'acide chlorhydrique;

(b) ladite solution de nitrite de sodium comprend 0,004 M de nitrite de sodium;

(c) le rapport de ladite solution d'acide sulfanilique à ladite solution de nitrite de sodium est préalablement choisi de manière à obtenir ladite solution diazoïque comprenant 0,004 M de colorant diazoïque;

(d) ledit processus de diazotation est poursuivi pendant 30 secondes;

(e) ladite partie aliquote dudit échantillon est introduite dans ladite solution diazoïque dans un rapport en volume de 1,55; et

(f) ladite mesure de la vitesse instantanée est effectuée 18 secondes après l'introduction de ladite partie aliquote dudit échantillon dans ladite solution diazoïque.

10. Procédé suivant la revendication 9, dans lequel ledit processus die diazotation est poursuivi à 37°C.

11. Procédé suivant la revendication 9, dans lequel ladite solution de nitrite de sodium comprend en outre un préservateur.

12. Procédé suivant la revendication 1, dans lequel:

(a) ladite solution d'acide sulfanilique comprend 0,03 M d'acide sulfanilique et 0,05 M d'acide chlorhydrique;

(b) ladite solution de nitrite de sodium comprend 0,004 M de nitrite de sodium et comprend en outre un préservateur;

(c) le rapport de ladite solution d'acide sulfanilique à ladite solution de nitrite de sodium est préalablement choisi de manière à obtenir ladite solution diazoïque comprenant 0,004 M de colorant diazoïque;

(d) ledit processus de diazotation est poursuivi pendant 30 secondes à 37°C;

(e) ladite partie aliquote dudit échantillon est introduite dans ladite solution diazoïque dans un rapport en volume de 1:55; et

(f) ladite mesure de la vitesse instantanée est réalisée 18 secondes après l'introduction de ladite partie aliquote dudit échantillon dans ladite solution diazoïque.